(19) Europäisches Patentamt
European Patent Office
Office européen des brevets

(11) **EP 4 323 757 B1**

(12) **EUROPEAN PATENT SPECIFICATION**

(45) Date of publication and mention
of the grant of the patent:
**11.06.2025 Bulletin 2025/24**

(21) Application number: **22723755.9**

(22) Date of filing: **31.03.2022**

(51) International Patent Classification (IPC):
**G01N 25/56** (2006.01)     **G01N 33/00** (2006.01)

(52) Cooperative Patent Classification (CPC):
**G01N 33/0036; G01N 25/56**

(86) International application number:
**PCT/IB2022/053008**

(87) International publication number:
**WO 2022/219443 (20.10.2022 Gazette 2022/42)**

(54) **A SYSTEM AND A METHOD FOR ESTIMATING MOISTURE CONTENT OF A GAS MIXTURE IN A SAMPLE HANDLING SYSTEM**

SYSTEM UND VERFAHREN ZUR SCHÄTZUNG DES FEUCHTIGKEITSGEHALTS EINER GASMISCHUNG IN EINEM PROBENHANDHABUNGSSYSTEM

SYSTÈME ET PROCÉDÉ D'ESTIMATION DE TENEUR EN HUMIDITÉ D'UN MÉLANGE GAZEUX DANS UN SYSTÈME DE MANIPULATION D'ÉCHANTILLONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.04.2021 IN 202141017407**

(43) Date of publication of application:
**21.02.2024 Bulletin 2024/08**

(73) Proprietor: **ABB SCHWEIZ AG**
**5400 Baden (CH)**

(72) Inventors:
• **MEKAPATI, Srnivas**
**Arundelpet, Andhra Pradesh 522002 (IN)**

• **ABHILASH, Pani**
**Bangalore, Karnataka 560037 (IN)**
• **SUBHASHISH, Dasgupta**
**Bangalore 560066 (IN)**
• **VINAY, Kariwala**
**Bangalore, Karnataka 560048 (IN)**
• **SABYASACHI, Bhattacharyya**
**Karyalay, Maharashtra Pune 411028 (IN)**

(74) Representative: **Zimmermann & Partner Patentanwälte mbB**
**Postfach 330 920**
**80069 München (DE)**

(56) References cited:
**EP-A2- 0 541 264      WO-A1-2020/053807**
**WO-A1-98/42434**

**Description**

**FIELD OF INVENTION**

The embodiment herein generally relates to a Sample Handling System (SHS). More particularly to a system and method for estimating a moisture content of a gas mixture in the SHS.

**BACKGROUND AND PRIOR ART**

Generally, a Sample Handling System (SHS) is deployed in an industry for analyzing, and processing/conditioning samples like gas mixture, fluid to improve efficiency of a process at an initial stage. Mostly, the SHS are used for filtrations, isolation and pressure/temperature/flow control at gas analyzer systems especially in industrial gas treatment, semiconductor, furnace gas and heat treatment.

When considering the SHS for gas analysis, a collection of the gas mixture comprising a flue gas from a pipeline has to undergo a purification process before delivering the gas mixture to a gas analyzer. The purification process generally takes place in the SHS. While, there are different designs of the SHS, the function of the SHS remains the same, which is to condition/purify the gas mixture to remove dust, moisture and corrosive substances from the gas mixture.

In the conditioning/purifying process of the gas mixture (gas samples), there are certain parameters that influence a performance of the SHS. In that, moisture content in the gas mixture is a crucial parameter that influences the performance of the gas analyzer in the SHS. In general, several types of equipment such as a gas cooler, a coalescence filter of thermoelectric condenser are being used conventionally to remove the moisture content from the gas mixture. However, it is not assured that a maximum volume of the moisture content is removed completely before sending to the gas analyzer. In order to address this issue, a hardware based moisture sensors are implemented in the SHS to monitor and estimate the moisture content of the gas mixture. The implementation of the hardware based moisture sensors increase cost of the SHS. Further, the implementation of the hardware based moisture sensors may require an undesirable change in a design of a conventional SHS. WO 2020/053807 A1 describes estimating a moisture content in a Sample Handling System, but not on a comparison of a calculated versus an actual surface area of the gas cooler.

Therefore, in light of the foregoing discussions, there is a need for a system and method for estimating the moisture content in the gas mixture without any additional cost.

**SUMMARY OF THE INVENTION**

It is an object of the present disclosure to mitigate, alleviate or eliminate one or more of the above-identified deficiencies and disadvantages in the prior art and solve at least the above-mentioned problem.

In view of the foregoing, the invention provides a system for estimating a moisture content of a gas mixture in a Sample Handling System and a method for estimating moisture content of a gas mixture in a Sample Handling System.

According to the invention, a system for estimating moisture content of a gas mixture in the Sample Handling System, SHS, provided, comprising a data pre-processing module and a moisture content estimation module. The data pre-processing module is configured to receive data comprising measurements of the SHS related to the gas mixture, a gas cooler and a coolant; and a plurality of parameters related to the gas mixture. The data pre-processing module is configured to analyze the received data to remove one or more anomalies in the data. The moisture content estimation module is configured to determine thermodynamic properties, heat transfer coefficient (U) and mass transfer coefficient (K) of the gas mixture based on the analyzed data. The moisture content estimation module is configured to calculate a surface area (A) of the gas cooler based on the analyzed data and the determined thermodynamic properties, heat transfer coefficient (U) and mass transfer coefficient (K) of the gas mixture. The moisture content estimation module is configured to estimate the moisture content of the gas mixture based on comparison of the calculated surface area (A) of the gas cooler with an actual surface area (CA) of the gas cooler.

According to an embodiment, the moisture content estimation module is configured to calculate the surface area of the gas cooler by determining initial temperatures (initial temperature profiles) of the gas mixture at the gas cooler of the SHS using computational fluid dynamics finite volume/finite element simulations. The flow of the gas mixture at an inlet of the gas cooler is predefined.

According to an embodiment, the moisture content estimation module is configured to estimate the moisture content of the gas mixture based on comparison of the calculated surface area of the gas cooler with the actual surface area of the gas cooler by determining that the calculated surface area (A) of the gas cooler matches with an actual surface area (CA) of the gas cooler. In response to the determination, the moisture content estimation module is configured to provide: an estimated flow of the gas mixture at the inlet of the gas cooler and condensate, an estimated temperature of the coolant at the outlet of the coolant and the moisture content of the gas mixture at the inlet and an outlet of gas cooler.

According to another embodiment, the moisture content estimation module is configured to iteratively perform the estimation of the thermodynamic properties, heat transfer coefficient (U) and mass transfer coefficient (K) of the gas mixture using the analyzed data with a modified value for the flow of the gas mixture at the inlet of the gas cooler, in response to the determination that the calculated surface area (A) of the gas cooler not matches with the actual surface area (CA) of the gas cooler.

According to an embodiment, the data-preprocessing module is further configured to analyze the data to remove one or more anomalies in the measurements of the SHS by assigning missing values of the measurements (M1-M6, S1) of the SHS, removing noise and outliers in the data using techniques for imputation, and filtering techniques.

According to an embodiment, the measurements of the SHS related to the gas mixture, the gas cooler and the coolant includes real time data from the gas cooler and the coolant of the SHS. The real time data includes pressure, temperature and flow of the gas mixture, the gas cooler and the coolant. The real time data from the gas cooler and the coolant of the SHS includes the temperatures and the flows at the inlet and the outlet of the gas cooler and at an inlet of the coolant.

According to an embodiment, the plurality of parameters related to the gas mixture comprises parameters for determining the thermodynamic properties of the gas mixture. The thermodynamic properties of the gas mixture includes specific heat, heat of condensation, saturated vapor pressure and dynamic viscosity

According to an embodiment, the plurality of parameters related to the gas mixture comprises a plurality of curves. The plurality of curves comprises Chilton curves. The Chilton curves provide Chilton factor based on Reynolds number of flow and the Chilton factor is utilized for determining the heat transfer coefficient (U) and mass transfer coefficient (K).

According to the invention, a method for estimating the moisture content in the gas mixture in the Sample Handling System, SHS, is provided. The method comprising the steps of: receiving data comprising measurements of the SHS related to the gas mixture, a gas cooler and a coolant; and a plurality of parameters related to the gas mixture; analyzing the received data to remove one or more anomalies from the data; determining thermodynamic properties, heat transfer coefficient (U) and mass transfer coefficient (K) of the gas mixture based on the analyzed data; calculating a surface area of the gas cooler based on the analyzed data and the determined thermodynamic properties, heat transfer coefficient (U) and mass transfer coefficient (K) of the gas mixture; and estimating the moisture content of the gas mixture based on comparison of the calculated surface area of the gas cooler with an actual surface area of the gas cooler.

**BRIEF** DESCRIPTION **OF DRAWINGS**

The detailed description is set forth with reference to the accompanying figures. In the figures, the left-most digit(s) of a reference number identifies the figure in which the reference number first appears. The use of the same reference numbers in different figures indicates similar or identical items.

Fig.1 illustrates a schematic of a conventional/existing Sample Handling System;

Fig. 2 illustrates an exemplary system for estimating moisture content of a gas mixture in the Sample Handling System, according to a first aspect of an embodiment herein;

Fig. 3 illustrates an exemplary method implemented for estimating the moisture content of the gas mixture in the Sample Handling System, according to a second aspect of an embodiment herein;

Fig. 4 illustrates an exemplary gas cooler of the Sample Handling System for introducing coolant, according to a first aspect of the embodiment herein; and

Figs. 5A and 5B illustrate an exemplary method depicting a sequence of steps performed for estimating moisture content of gas mixture in the Sample Handling System, according to a second aspect of the embodiment herein.

**DETAILED DESCRIPTION OF THE PREFERRED EMBODIMENTS**

The embodiments herein and the various features and advantageous details thereof are explained more fully with reference to the non-limiting embodiments and detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

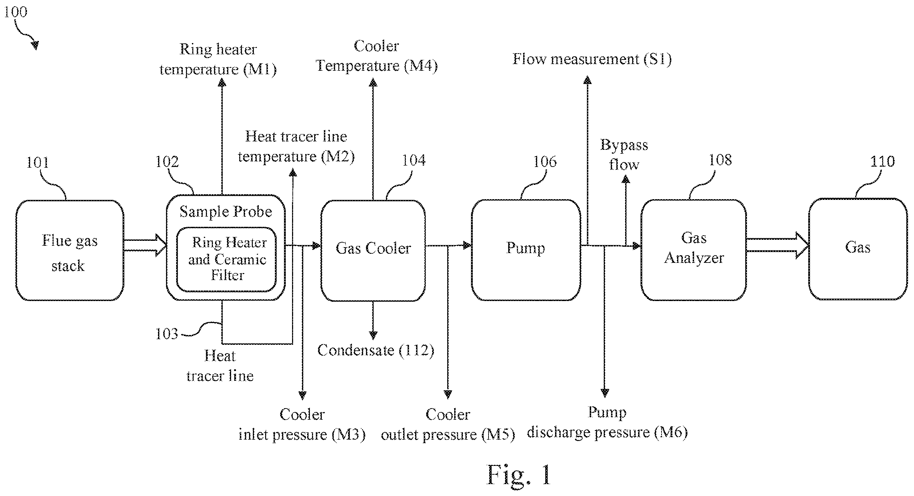

As mentioned above, there is a need for a system and a method for estimating moisture content in a gas mixture comprising a flue gas and water vapor by making use of measurements of SHS without any additional cost. The embodiments herein achieve this by providing a system and a method for estimating the moisture content in the gas mixture comprising the flue gas (exhaust gas or stack gas) and water vapor. Referring now to the drawings, and more particularly to FIGS. 1 through 5B, where similar reference characters denote corresponding features consistently throughout the figures, there are shown preferred embodiments.

Fig. 1 illustrates a schematic of a conventional/ existing Sample Handling System (SHS) 100.The conventional SHS 100 includes a plurality of components to purify a gas mixture comprising a flue gas and water vapor before the gas mixture is analyzed at a gas analyzer. The SHS 100 may be implemented in power plants, refineries, petrochemical and chemical plants, among others. The Sample Handling Systems, SHS, are used for isolation, filtration, and pressure/-temperature/flow control of analyzer systems especially implemented in industrial gas treatment, furnace gas and heat treatment, semiconductor fabrication, power generation, air dryer and pharmaceutical applications. While, there may be different types and designs of commercially available SHS, the function of all types of SHS remains the same, which is to condition the gas mixture to remove dust, moisture and corrosive substances from the gas mixture. The gas mixture is obtained from a flue gas stack 101 and is passed through a pipe or a flue to exit into an atmosphere. While the gas mixture passes through the pipe/pipeline, the gas mixture is conditioned in the SHS 100 using a number of components of the SHS 100 to remove the moisture from the gas mixture. The term "conditioned" herein may correspond to exposing the gas mixture to a certain temperature to heat it and cool it using a coolant.

The flue gas comprised in the gas mixture refers to the gas being released into the atmosphere via the flue, which is the pipe or a channel for conveying exhaust gases from industrial plants. A composition of the flue gas depends on material that is burned. However, the composition of the flue gas usually comprises of nitrogen derived from the combustion of air, carbon dioxide, water vapor as well as oxygen. The composition of the flue gas further comprises a small percentage of a number of pollutants, such as particulate matter, carbon monoxide, nitrogen oxides, and sulfur oxides. The different measurements are obtained at various gas-mixture processing stages of the SHS 100 and the various gas mixture processing stages of the SHS 100 are described below.

The SHS 100 includes the flue gas stack 101 from where the gas mixture comprising the flue gas and the water vapor is fed into the SHS 100. The SHS 100 further includes a sample probe 102 and a heat tracer line 103. The sample probe 102 and the heat tracer line 103 comprising a ring heater and a ceramic filter as integral parts. The SHS 100 further includes a gas cooler 104, a sample pump 106 and a gas analyzer 108. Upon being analyzed, the gas mixture provides various gases 110 as an output that are present in the composition of the flue gas as described earlier.

The gas mixture is taken into the SHS 100 via the sample probe 102. The gas mixture is filtered to remove dust/colloidal particles using a ceramic filter and is heated by the ring heater of the sample probe 102 and the heat tracer line 103. Temperature of the ring heater is maintained at a value "M1" and temperature of the heat tracer line 103 is maintained at a value "M2". By maintaining the temperatures of the ring heater at M1 and the heat tracer line 103 at M2, the gas mixture that passes from the sample probe 102 to the gas cooler 104 is heated. The heating is performed to maintain a desired temperature of the gas mixture until the gas mixture reaches the gas cooler 104. This is required to avoid condensation of the moisture in the pipeline which otherwise creates several operational problems.

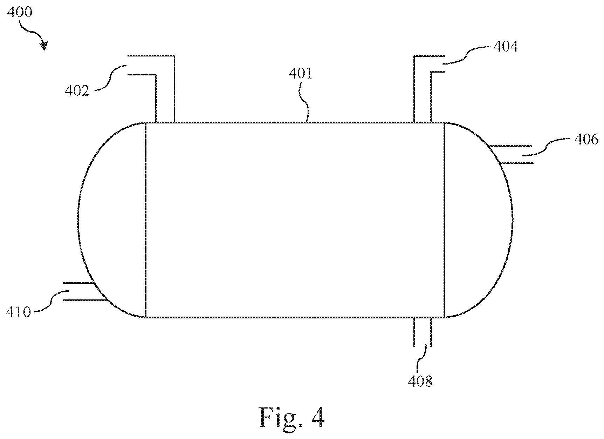

The coolant being introduced into the gas cooler through an inlet of the coolant (shown in Fig.4), which is different from an inlet of the gas cooler (shown in Fig.4) through which the heated gas mixture enters the gas cooler 104. The gas cooler 104 and its various inlets and outlets are described in-detail in reference with Fig. 4. Once the heated gas mixture reaches the gas cooler 104, the coolant that has entered the gas cooler 104 removes the heat from the gas mixture. This removal of heat from the gas mixture is resulted in condensation of the moisture and that is removed as condensate 112. The dry gas mixture is then pumped via the sample pump 106 to the gas analyzer 108 for analyzing the various gases.

The various gases are the gases being released into the atmosphere as an output 110 and are composed of gases such as nitrogen ($N_2$), carbon dioxide ($CO_2$), oxygen ($O_2$). It also includes water vapor as well as small percentage of pollutants, carbon monoxide (CO), nitrogen oxides (NO, $NO_2$, etc.,) , sulfur oxides (SO, $SO_2$ etc.,) and so on.

The SHS 100 is configured to perform measurements (M1-M6 , S1) related to the gas mixture, the gas cooler and the coolant at various inlets, outlets and bodies of the various gas mixture processing stages of the SHS 100 and generate data corresponding to the measurements. For example, temperature and pressure are measured at an inlet and an outlet of the gas cooler 104. Temperature within the gas cooler 104 and pump discharge pressure is measured. This measured data is a real time data. The measurements of the SHS related to pressure, temperature and flow of the gas mixture, the gas cooler and the coolant includes the real time data obtained from the plurality of measurements. The real time data includes the ring heater temperature M1, the heat tracer line temperature M2, a pressure M3 at the inlet of the gas cooler 204, a temperature M4 at the gas cooler 204, a pressure M5 at the outlet of the gas cooler 204, the pump discharge pressure M6 and a flow measurement S1, among other measurements.

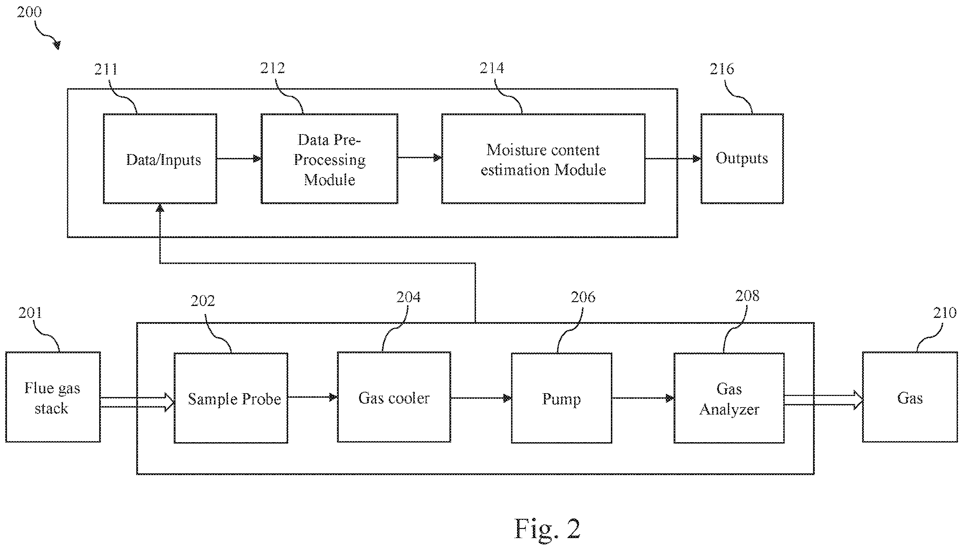

Some or all of these real time data can be used for estimation of the moisture content in the gas mixture. The estimation of the moisture content in the gas mixture is performed using one or more modules where the real time data and other data can be pre-processed and the moisture content in the gas mixture is estimated. The estimation of the moisture content in the gas mixture and the modules used for the estimation are described in the Figs 2-5.

Fig. 2 illustrates a system 200 for estimating moisture content of a gas mixture in a Sample Handling System (SHS), according to a first aspect of an embodiment herein. The SHS may be the exemplary SHS 100 described in reference to Fig. 1. The system 200 includes a data pre-processing module 212 and a moisture content estimation module 214. The data pre-processing module 212 and the moisture content estimation module 214 may be modules independent from the SHS 100. The system 200 further includes the various gas mixture conditioning stages of the SHS 100. The various conditioning stages of the SHS includes a flue gas stack 201, a sample probe 202, a gas cooler 204, a sample pump 206 and a gas analyzer 208. The various gases 210 are being released into the atmosphere as described earlier. The flue gas stack 201, a sample probe 202, a gas cooler 204, a sample pump 206 and a gas analyzer 208 may be same as the sample probe 102, the gas cooler 104, the sample pump 106 and the gas analyzer 108 as described in reference with Fig. 1 and are not repeated for the sake of brevity. Although not shown, one could infer that the system 200 comprises a ring heater and a ceramic filter within the sample probe 202 as integral parts of the sample probe 102 and a heat tracer line similar to the heat tracer line 103.

Alternatively, the data pre-processing module 212 and the moisture content estimation module 214 may be discrete and separate modules placed remotely from each other. The data pre-processing module 212 and the moisture content estimation module 214 may include one or more processors, a memory and communication interfaces to communicate data between the data pre-processing module 212 and the moisture content estimation module 214. For example, the data pre-processing module 212 is configured to provide the analyzed data for the estimation of the moisture content of the gas mixture to the moisture content estimation module 214 via one or more of the communication interfaces.

Examples of communication interfaces may include one or more inter or intra system buses such as USB and SPI, among others. Such communication interfaces may be implemented when the data pre-processing module 212 and the moisture content estimation module 214 are within a single device. Examples of communication interfaces may also include, wireless communication network and wired communication networks. Such communication interfaces may be implemented when the data pre-processing module 212 and the moisture content estimation module 214 are remotely placed from one another. Since, the data pre-processing module 212 and the moisture content estimation module 214 are independent and not a part of the SHS 100, this provides a solution for estimating moisture content without additional hardware components being added to the SHS 100 that could increase the cost of the SHS 100.

According to the embodiment, the data pre-processing module 212 is configured to receive data 211. The data 211 includes measurements (M1-M6, S1) of the SHS 100 related to the gas mixture, the gas cooler and the coolant, and a plurality of parameters related to the gas mixture comprising the flue gas and water vapor. The measurements (M1-M6, S1) of the SHS includes real time data from the gas cooler (104, 204) and the coolant. The real time data from the gas cooler 204 and the coolant includes temperatures and flows of a gas cooler 204 and a coolant. The temperatures and the flows of the gas cooler 204 and the coolant includes the temperature of the gas cooler 204 at an inlet (shown in Fig. 4) and an outlet (shown in Fig. 4), the flow of the gas cooler 204 at the outlet, the temperature at an inlet (shown in Fig. 4) of the coolant and the flow of the coolant at the inlet (shown in Fig. 4).

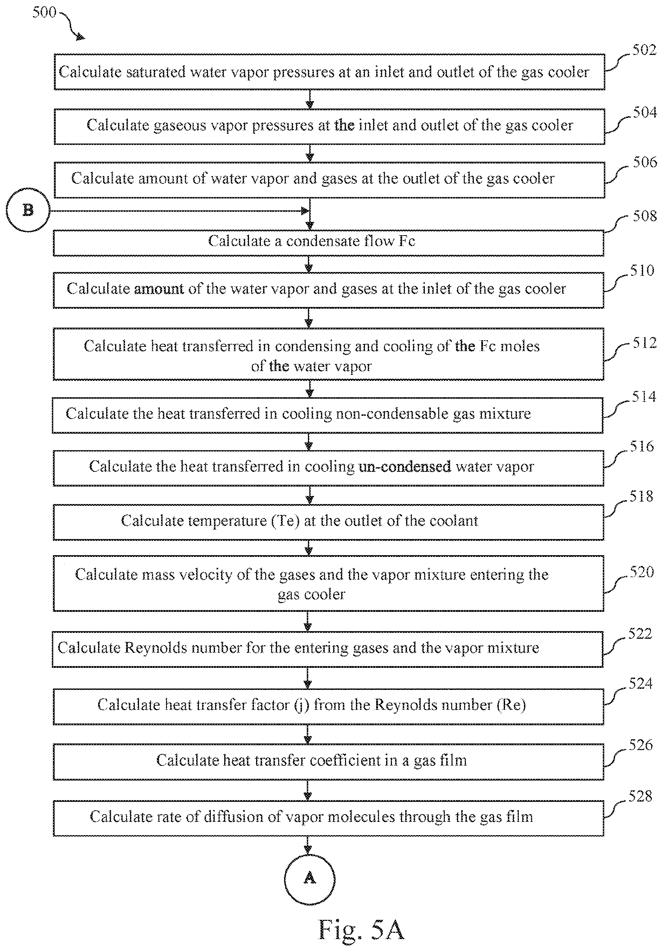

The plurality of parameters related to the gas mixture comprises parameters for determining thermodynamic properties of the gas mixture. The thermodynamic properties of the gas mixture includes parameters, which include coefficients that are used in a set of equations (described in FIG.5A-5B) to estimate thermodynamic properties of the compositions of the flue gas in the gas mixture. For example, the coefficients includes but is not limited to heat transfer coefficient (H), a mass transfer coefficient (M) and so on. The thermodynamic properties of the gas mixture includes, but are not limited to, specific heat, heat of condensation, saturated vapor pressure and dynamic viscosity.

The real time data further includes a pressure (M3) at the inlet of the gas cooler 204, pressure (M5) at the outlet of the gas cooler 204 and a pump discharge pressure (M6). The plurality of curves includes Chilton curves (not shown). The Chilton curves provide Chilton factor based on Reynolds number of flow (Re).

The data pre-processing module 212 is configured to analyze the received data to remove one or more anomalies from the data 211. The data 211 is analyzed for any missing values measurements (M1-M6, S1) of the SHS 100, noise and outliers in the data 211. The data-preprocessing module 212 is further configured to assign the missing values in the measurements (M1-M6, S1) of the SHS 100 using known techniques for imputation, and remove the noise and outlier in the data 211 using known filtering techniques based on the type of application. For example, upon receiving the data 211, the data is analyzed for any missing values in the measurements (M1-M6, S1) of the SHS 100 and then those missing values may be imputed. A technique herein may be defined as a set of algorithms or instructions that define the manner in which a particular task needs to be performed. For example, the technique for imputation may correspond to a set of algorithms or instructions that define the manner in which imputation needs to be performed. Likewise, the filtering technique may correspond to a set of algorithms or instructions that define the manner in which filtering needs to be performed. An example of the techniques for imputation include, but is not limited to, moving averages filter technique. An example of the filtering techniques include, but is not limited to Fast Fourier Transformation. The assigned missing values and the noise and outlier free data may be referred to as the analyzed data. The data pre-processing module 212 is configured to provide the analyzed data for the estimation of the moisture content of the gas mixture.

According to a first aspect of the embodiment, the moisture content estimation module 214 is configured to determine the thermodynamic properties, heat transfer coefficient (U) and mass transfer coefficient (K) of the gas mixture based on the analyzed data provided by the data pre-processing module 212. The moisture content estimation module 214 is further configured to calculate a surface area (A) of the gas cooler 204 based on the analyzed data and the determined thermodynamic properties, the heat transfer coefficient (U) and the mass transfer coefficient (K) of the gas mixture. The moisture content estimation module 214 is further configured to estimate the moisture content of the gas mixture based on comparison of the calculated surface area (A) of the gas cooler (104, 204) with an actual (known) surface area (CA) of the gas cooler.

According to a first aspect of the embodiment, the moisture content estimation module 214 is configured to calculate the surface area of the gas cooler 204 using the analyzed data by balancing/ stabilizing mass and heat transfer at different condensate temperatures in the gas cooler 204. The calculated surface area (A) of the gas cooler 204 can be used to estimate the moisture content of the gas mixture at the inlet and outlet of the gas cooler 204 in the SHS 100.

According to the first aspect of the embodiment, the moisture content estimation module 214 is configured to calculate the surface area (A) of the gas cooler (104, 204, 400) by determining initial temperatures (initial temperature profiles) of the gas mixture at the inlet and the outlet of the gas cooler 204 in the SHS 100. The estimation of the initial temperature profiles of the gas mixture is performed by solving equations (described in FIG.5A-5B) for conservation of mass, momentum and energy. The estimation of the initial temperature profiles are performed using computational fluid dynamics finite volume/finite element, CFD, simulations in three dimension. The flow of the gas mixture at the inlet of the gas cooler is predefined. The initial temperature profiles of the gas mixture is used to calculate the surface area (A) of the gas cooler 204.

According to the embodiment, the moisture content estimation module 214 is configured to estimate the moisture content of the gas mixture based on comparison of the calculated surface area of the gas cooler (104, 204, 400) with the actual surface area of the gas cooler (104, 204, 400). The calculated surface area of the gas cooler is compared with the actual surface area of the gas cooler by determining that the calculated surface area (A) of the gas cooler 204 matches with the actual surface area (CA) of the gas cooler 204. In response to the determination, the moisture content estimation module is configured to provide: an estimated flow of the gas mixture at the inlet of the gas cooler and condensate 112, an estimated temperature of the coolant at the outlet and the moisture content of the gas mixture at the inlet 402 and the outlet 404 of the gas cooler 204.

According to another embodiment, the moisture content estimation module 214 is configured to iteratively perform the estimation of the thermodynamic properties, heat transfer coefficient (U) and mass transfer coefficient (K) of the gas mixture using the analyzed data with another predefined value for the flow of the gas mixture at the inlet of the gas cooler, in response to the determination that the calculated surface area (A) of the gas cooler (104, 204) not matches with the actual surface area (CA) of the gas cooler (104, 204) the moisture content estimation module. In order to calculate the surface area of the gas cooler 204, a sequence of calculations are performed. The sequence of calculations are formulated in an iterative way to solve a set of equations in a simplified manner. The sequence of calculations are described later with reference to Fig. 5A-5B.

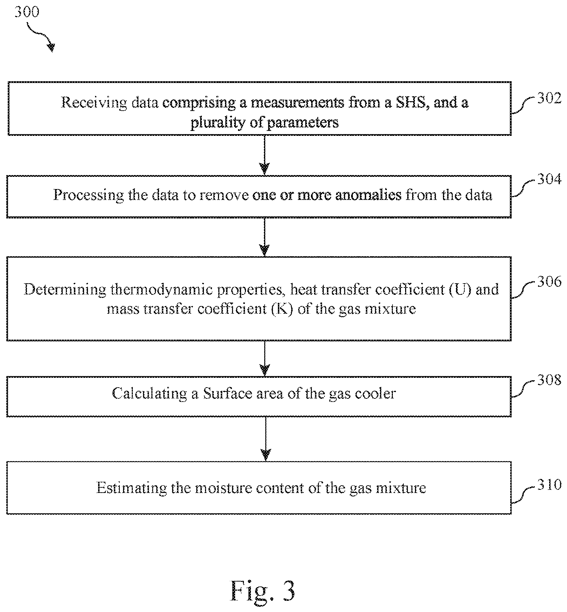

Fig. 3 illustrates an exemplary method 300 implemented for estimating moisture content of the gas mixture in the SHS 100, according to a second aspect of an embodiment. The methods that are illustrated in Figs. 3 and 5A-5B, as a collection of operations in a logical flow graph representing a sequence of operations that can be implemented in hardware, software, firmware, or a combination thereof. In the context of software, the operations represent computer instructions that, when executed by one or more processors, perform the recited operations. The various method steps performed by each of the data pre-processing module 212 and the moisture content estimation module 214 for estimating moisture content of a gas mixture in the SHS 100 may occur in a sequential manner.

At step 302, the data 211 is received by the data pre-processing module 212. The data 211 may be received through one or more input devices from user/operator and stored in a memory. The data 211 includes measurements (M1-M6, S1) of the SHS related to the gas mixture, a gas cooler and a coolant, and a plurality of parameters related to the gas mixture. The measurements (M1-M6, S1) includes at least real time data from the gas cooler. The plurality of parameters related to the gas mixture comprises parameters for determining thermodynamic properties of the gas mixture. The thermodynamic properties of the gas mixture includes specific heat, heat of condensation, saturated vapor pressure and dynamic viscosity. The gas mixture comprising the flue gas and water vapor.

The measurements (M1-M6, S1) of the SHS related to the gas mixture, the gas cooler and the coolant includes a real time data obtained from the SHS. The real time data is related to pressure, temperature and flow of the gas mixture, the gas cooler and the coolant. The real time data includes the temperature of the gas cooler 204 at an inlet and an outlet of the gas cooler, the flow of the gas cooler 204 at the outlet, the temperature at an inlet of the coolant and the flow of the coolant at the inlet. The real time data further includes a pressure (M3) at the inlet of the gas cooler 204, pressure (M5) at the outlet of the gas cooler 204 and a pump discharge pressure (M6). The plurality of curves comprises Chilton curves, wherein the Chilton curves provide Chilton factor based on Reynolds number of flow, wherein the Chilton factor is utilized for determining the heat transfer coefficient (U) and mass transfer coefficient (K).

At step 304, the received data is analyzed to remove one or more anomalies from the data 211. The received data is processed by assigning/imputing missing values in the measurements (M1-M6, S1) of the SHS and removing noise and outliers in the data 211. The missing values in the measurements (M1-M6, S1) of the SHS are assigned/imputed using known techniques for imputation, such as moving averages filter technique. Noises and outliers are removed from the data 211 using known filtering techniques such as Fast Fourier Transformation. The imputed data and the noise and outlier free data may be referred to as the processed data. The analyzed data is provided for the estimation of the moisture content of the gas mixture.

At step 306, the thermodynamic properties, heat transfer coefficient (U) and mass transfer coefficient (K) of the gas mixture are determined using the analyzed data provided by the data pre-processing module 212.

At step 308, a surface area (A) of the gas cooler is calculated based on the analyzed data and the determined thermodynamic properties, heat transfer coefficient (U) and mass transfer coefficient (K) of the gas mixture.

At step 310, the moisture content of the gas mixture at the gas cooler inlet and the gas cooler outlet of the gas cooler 104, 204 in the SHS 100 is calculated based on comparison of the calculated surface area (A) of the gas cooler (104, 204) with an actual surface area (CA) of the gas cooler (104, 204). The calculated surface area (A) of the gas cooler 104, 204 can be used to estimate a percentage of the moisture content of the gas mixture at the inlet and outlet of the gas cooler 104, 204 in the SHS 100.

Fig. 4 illustrates an exemplary gas cooler 400 of the SHS 100 for introducing a coolant, according to a first aspect of an embodiment herein. The gas cooler 400 includes a cooler body 401 and a plurality of inlets and outlets (402-410). The plurality of inlets and outlets includes an inlet 402 and an outlet 404 of the gas cooler and an inlet 406 and an outlet 410 of the coolant and a water outlet 408.

The gas mixture enters the gas cooler 400 through the inlet 402 and simultaneously the coolant enters the gas cooler 400 through the inlet 406 of the coolant. The gas mixture, as described above, is heated using the ring heater in the sample probe 102 and the heat tracer line 103 before entering the gas cooler 400. The heated gas mixture exchanges heat with the coolant by conduction through the gas cooler body 401. The gas mixture may exchange the heat with the coolant and the water vapor comprised in the gas mixture loses the heat. The water vapor in the gas mixture starts condensing into a pure/clean liquid. The pure liquid that is saturated may leave through the water outlet 408. The coolant absorbs heat from the heated gas mixture and temperature of the coolant is increased. The coolant that absorbs the heat may leave the gas cooler 400 through the outlet 410 of the coolant. The gas mixture with a remaining water vapor leaves the gas cooler 400 through the outlet 404. Condensation of the water vapor occurs in a presence of non-condensable gases inside the gas cooler 400. The amount of a moisture in the gas mixture at the inlet 402 and outlet 404 of the gas cooler can be estimated at the moisture content estimation module 214 using the sequence of calculations that are formulated to solve a set of equations.

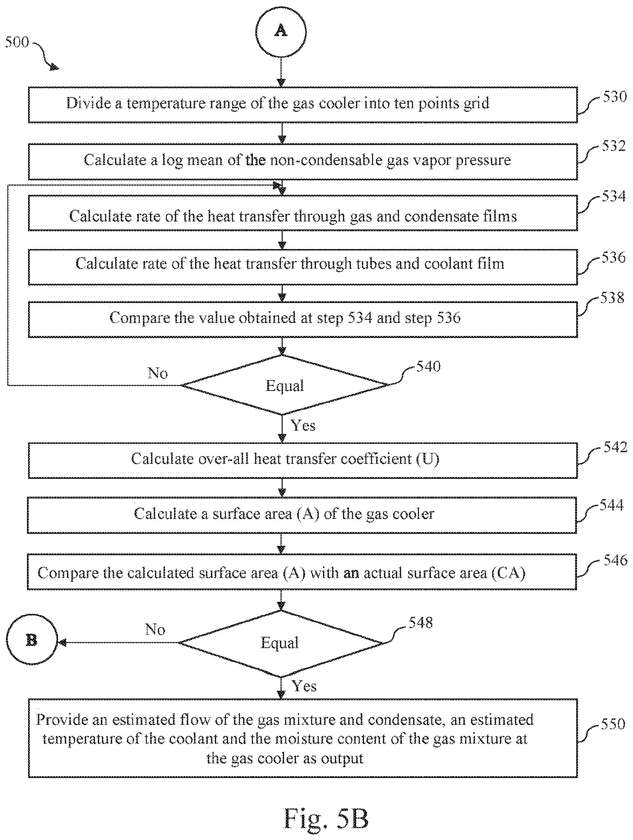

Figs. 5A and 5B illustrate an exemplary method 500 depicting a sequence of steps performed for estimating a moisture content of a gas mixture in a Sample Handling System, SHS, according to a second aspect of an embodiment herein. The method 500 illustrates steps (502-550) representing the sequence of calculations that are formulated to solve a set of equations.

At step 502, saturated water vapor pressures ($p_{v,i}$ and , $p_{v,o}$) at an inlet 402 and an outlet 404 of a gas cooler are calculated from given temperatures using Clausius-Clapeyron equation or the equation given below.

$$P_v = e^{\left(18.79 - 0.0075*T - \frac{5965.6}{T}\right)} \qquad \text{-------(1)}$$

where T is the temperature in Kelvin

The given temperature, could for example, include the temperatures and flows of the gas cooler 104, 204, 400 and a coolant comprises: the temperature of the gas cooler 104, 204, 400 at the inlet 402 and the outlet 404 of the gas cooler, the flow of the gas cooler 104, 204, 400 at the outlet 404, the temperature at the inlet 406 of the coolant and the flow of the coolant at the inlet 406.

At step 504, gaseous (i.e., non-condensable flue gases) vapor pressures at the inlet 402 and the outlet 404 of the gas cooler are calculated using saturated water vapor pressures ($p_{v,i}$ and $p_{v,o}$) at the inlet 402 and the outlet 404 of the gas cooler using a following equation.

$$p_{g,i} = 1 - p_{v,I}$$
$$p_{g,o} = 1 - p_{v,o} \qquad \text{-(2)}$$

where $p_{g,i}$ is the gaseous vapor pressures at the gas cooler inlet 402; and
$p_{g,o}$ is the gaseous vapor pressures at the gas cooler outlet 404.

At step 506, an amount/ quantities or moles of the water vapor and gases at the outlet 404 of the gas cooler is calculated using a flow of the gas mixture at the outlet 404 of the gas cooler. The gas mixture comprising gas (flue gas) and water vapor (vapor mixture).

Moles of the water vapor leaving the outlet 404 of the gas cooler, $W_b = F_b * p_{v,o}$
Moles of gases leaving the outlet 404 of the gas cooler,

$$G_b = F_b * p_{g,o} \quad -(3)$$

where $F_b$ = Flow of gas and vapor mixture at the outlet 404 of the gas cooler; and $p_{g,o}$ = gaseous vapor pressure at the outlet 404 of the gas cooler.

At step 508, a condensate flow ($F_c$) is calculated by assuming the flow of the gas mixture ($F_a$, should be greater than exit flow at the outlet 404 of the gas cooler) at the inlet 402 of the gas cooler using the following equation.

$$F_c = F_a - F_b \qquad -(4)$$

Where, $F_c$ = the condensate flow
$F_a$ = the flow of the gas mixture at the inlet 402 of the gas cooler
$F_b$ = the flow of the gas mixture at the outlet 404 of the gas cooler

At step 510, amount or quantities or moles of the water vapor and gases at the inlet 402 of the gas cooler ($W_a$) is calculated using the water vapor pressure ($p_{v,i}$) and gases vapor pressure ($p_{g,i}$)

Moles of the water vapor entering the inlet 402 of the gas cooler $W_a = F_a * p_{v,i}$
Moles of gases entering the inlet 402 of the gas cooler

$$G_a = F_a * p_{g,i} \qquad -(5)$$

At step 512, heat transferred in condensing and cooling of the $F_c$ moles of the water vapor is calculated using a following equation.

$$\Delta H_1 = F_c * (LH_{w,a} + CP_{w,a} * (T_a - T_b)) \quad -(6)$$

where $LH_{w,a}$ = latent heat of the water vapor at temperature $T_a$
$CP_{w,a}$ = specific heat of the water vapor at the temperature $T_a$
$T_a$ = the temperature at the inlet 402 of the gas cooler

At step 514, the heat transferred in cooling the non-condensable gas (flue gas or gaseous) mixture is calculated using a following equation.

$$\Delta H_2 = G_a * CP_{g,a} * (T_a - T_b)) \qquad -(7)$$

where $CP_{g,a}$ = specific heat of the non-condensable gases at the temperature $T_a$
At step 516, the heat transferred in cooling un-condensed water vapor is calculated using a following equation

$$\Delta H_3 = (W_a - F_c) * CP_{w,a} * (T_a - T_b)) \qquad -(8)$$

and then a total heat transferred to coolant is calculated using a following equation.

$$\Delta H = \Delta H_1 + \Delta H_2 + \Delta H_3 \qquad -(9)$$

At step 518, temperature ($T_e$) at the outlet (410) of the coolant is calculated by equating the total heat transferred ($\Delta H$) to the coolant temperature rise.

$$\Delta H = F_d * (T_d - T_e) \qquad -(10)$$

where $T_d$ = temperature of the inlet 406 of the coolant

$T_e$ = the temperature of the outlet 410 of the coolant

At step 520, mass velocity of the gases and the vapor mixture entering the gas cooler/a heat exchanger with known a cross-sectional area say "CA" is calculated.

$$\text{Mass velocity}, MV = \rho * v = \frac{(G_a - W_a)}{CA} \qquad -(11)$$

At step 522, Reynolds number for the entering gases and the vapor mixture is calculated. Subsequently or simultaneously, the viscosity ($\mu$) of the gas mixture from the temperature ($T_a$) at the inlet 402 of the gas cooler 204 is calculated.

$$\text{Reynolds Number}, Re = \frac{(D * MV)}{\mu} \qquad - (12)$$

At step 524, heat transfer factor (j) from the Reynolds number (Re) is calculated using the plot given in Chilton and Colburn (1934). Alternatively, an approximate function/curve can be used to calculate the heat transfer factor (j) from the Reynolds Number (Re) calculated at step 522.

$$j = f(Re) \qquad - (13)$$

At step 526, heat transfer coefficient (Colburn, 1934) in a gas film (the water vapor and the gases mixture) is calculated using a following equation.

$$h_s = j * c * \frac{MV}{\left(\frac{c\mu}{k}\right)^{\frac{2}{3}}} \qquad - (14)$$

where j = the heat transfer factor from step 524
c = specific heat at constant pressure
MV = the mass velocity calculated in step 520
$(c\mu/k)^{2/3}$ = 0.83 (Colburn, 1934)

At step 528, rate of diffusion of vapor molecules through the gas film (Chilton and Colburn, 1934) is calculated using a following equation.

$$K = j * \frac{MV}{M_m * p_{gf}\left(\frac{\mu}{\rho K_d}\right)^{\frac{2}{3}}} \qquad - (15)$$

where j = the heat transfer factor is obtained from step 524
MV = the mass velocity is calculated in step 520
$M_m$ = Average molecular weight of the gas mixture
$p_{gf}$= Log mean of the non-condensable gas vapor pressure between the main body ($p_g$) and adjacent to a condensate surface ($p_{g'}$)
$(\mu/\rho k_d)^{2/3}$ = 0.71 (Colburn, 1934).

At step 530, temperature range of the gas cooler (104, 204, 400) is divided into a ten point grid and a heat transfer coefficient value is assigned to the divided ten points grid of the temperature range of the gas cooler (104, 204, 400). For assigning a first value in the ten points grid, the temperature range is divided into the ten points for a particular range of temperature. For example, if the temperature range is between 80 and 60, then the range is divided as 80, 78... 60. Subsequently, at each temperature (i.e., 80, 78...) the condensate temperature ($t_c$) is assumed and iterative calculations (from 532 to 538) are repeated for a heat balance. If it is balanced or stabilized, then the assumed condensate temperature ($t_c$) is right. If it is not balanced, new value for the condensate temperature ($t_c$) is assumed and from step 532 to step 538 are repeated.

At step 532, the log mean of the non-condensable gas vapor pressure ($p_{gf}$) is calculated by assuming a temperature of a condensate surface ($t_c$) using a following equation.

$$h_s * (t_g - t_c) + K * \lambda * (p_v - p_c) = h_0 * (t_c - t_w) \qquad -(16)$$

where $h_s$ = sensible heat transfer coefficient calculated from step 526
$t_g$ = non-condensable gas temperature (equal to $T_a$)
$t_c$ = condensate surface temperature (to be estimated from equation 16)
K = the mass transfer coefficient calculated from the step 528 (as "K=constant/$p_{gf}$" from equation 15)
$\lambda$ = latent heat of water vapor (to be calculated based on $T_a$)
$p_v$ = the water vapor pressure at mixture temperature (to be calculated from Clausius Clapeyron equation for $T_a$)
$p_c$ = the water vapor pressure at condensate surface temperature (to be calculated from the estimate of $t_c$ using Clausius Clapeyron equation)

The temperature of a condensate surface $t_c$ is assumed based on which $p_c$, $p_g$', $p_{gf}$ are calculated and substituted in equation 16. It shall be noted that $t_c$, $p_{gf}$ are estimated iteratively in the equation (16) till the balance is reached. That is, the value in the left hand side of the equation equals to the value in the right hand side of the equation.

At steps 534 and 536, the left hand side (i.e. rate of heat transfer through gas and condensate films) and the right hand side (i.e. rate of heat transfer through tubes provided inside the gas cooler body 401 and coolant film) of the equation (16) are calculated. As already described, $t_c$ and $p_{gf}$ are estimated iteratively in the equation (16) till the value in the left hand side of the equation (16) equals to the value in the right hand side. The iteration stops when finally the value in the left hand side of the equation (16) equals to the value in the right hand side. The value of the rate of the heat transfer through gas and condensate films and the rate of the heat transfer through the tubes of the gas cooler body 401 and coolant film are the values at which the iteration stops.

At step 538, a value of the rate of the heat transfer through gas and the condensate films is compared to a value of the rate of the heat transfer through the tubes of the gas cooler body 401 and the water film.

At step 540, as a result of comparison it is determined that if the value of the rate of the heat transfer through gas and condensate films is equal to the value of the rate of the heat transfer through the tubes of the gas cooler body and water film. If the values are equal, then the method 500 proceeds to step 542. If it is determined that the values are not equal, then the method 500 repeats the calculations from step 532 to step 540.

At step 542, over-all heat transfer coefficient (U) is calculated by equating the heat transfer through the gas film and latent heat using the following equation.

$$h_s * (t_g - t_c) + K * \lambda * (p_v - p_c) = U * \Delta t \qquad -(17)$$

where $\Delta t = T_a - T_e$
$h_s$ = film coefficient of heat transfer
U = the overall coefficient of heat transfer

At step 544, value of the overall heat transfer coefficient (U) calculated at step 542 is now assigned as the first value in the grid.

To calculate a surface area (A) of the gas cooler (104, 204, 400) the parameters $t_g$, $t_w$, $\Delta t$, $t_c$, $p_g$, $p_{gf}$, q, m, Re, K and U may be used. Where '$t_g$' refers to the non-condensable gas temperature, '$t_w$' refers to water temperature, '$\Delta t$' refers to temperature difference between the temperature at the inlet 402 of the gas cooler 400 and the outlet 410 of the coolant, and '$t_c$' refers to the temperature of condensate surface. Where '$p_g$' refers to non-condensable gas pressure, '$p_g$"' refers to vapor pressure adjacent to the condensate surface, '$p_{gf}$' refers to the log mean temperature of '$p_g$' and '$p_g$"', q refers to heat transferred, m refers to the mass flow, 'Re' refers to the Reynolds number, K refers to the mass transfer coefficient, and 'U' refers to over-all heat transfer coefficient. $t_g$, $t_w$, $\Delta t$, $t_c$ may collectively form the initial temperature profiles or initial temperatures.

For calculation of the surface area of the gas cooler (104, 204, 400), several temperatures between $T_a$ and $T_b$ across the length of the condenser or the gas cooler (104, 204, 400), say ten points are identified. The condensate surface temperature ($t_c$) and the overall heat transfer coefficient (U) are calculated by iterating the equation 16 of the step 532 and equation 17 of the step 542 at all the 10 intervals between $T_a$ and $T_b$. This results into the following table, Table 1 (say $T_a$ is 90°C and $T_b$ is 50°C).

Table 1

| Point | 1 | 2 | 3 | 4 | 5 | 6 | 7 | 8 | 9 | 10 |
|---|---|---|---|---|---|---|---|---|---|---|
| $t_g$(°C) | 90 | - | - | - | - | - | - | - | - | 50 |

(continued)

| Point | 1 | 2 | 3 | 4 | 5 | 6 | 7 | 8 | 9 | 10 |
|---|---|---|---|---|---|---|---|---|---|---|
| $t_w$(°C) | - | - | - | - | - | - | - | - | - | - |
| $\Delta t$ (°C) | - | - | - | - | - | - | - | - | - | - |
| $t_c$(°C) | - | - | - | - | - | - | - | - | - | - |
| $p_g$(atm) | - | - | - | - | - | - | - | - | - | - |
| $p_{gf}$(atm) | - | - | - | - | - | - | - | - | - | |
| q(p.c.u/h) | - | - | - | - | - | - | - | - | | |
| m(lb/h) | - | - | - | - | - | - | - | - | - | - |
| Re | - | - | - | - | - | - | - | - | - | - |
| K | - | - | - | - | - | - | - | - | - | - |
| U | - | - | - | - | - | - | - | - | - | - |
| $U\Delta t$ | - | - | - | - | - | - | - | - | - | - |
| A(sq.ft.) | - | - | - | - | - | - | - | - | - | |

The surface area (A) of the gas cooler (104, 204, 400) is calculated based on the area under a curve (not shown) of "q vs (1/U$\Delta$t)", where q refers to heat transferred, 'U' refers to overall heat transfer coefficient and $\Delta$t refers to temperature difference between temperature at the inlet 402 of the gas cooler and the outlet 410 of the coolant are shown in the Table 1 above. Each value of 'q' against each value of 'U$\Delta$t' between points 1 to 10 are shown in the Table 1. A graph (curve) (not shown) can be obtained by combining these values of 'q' against 'U$\Delta$t'.

At step 546, the calculated surface area (A) of the gas cooler (104, 204, 400) at step 544 is compared with the actual surface area (CA) of the gas cooler (104, 204, 400).

At step 548, it is determined whether the calculated surface area (A) of the gas cooler (104, 204, 400) matches the actual surface area (CA) of the gas cooler (104, 204, 400). If it is determined that the calculated surface area (A) of the gas cooler (104, 204, 400) and the actual surface area (CA) of the gas cooler 104, 204, 400 matches or almost equal or very close (considerable difference between the CA and C or very less difference), then the method 500 proceeds to step 550. Further, the estimated moles of water vapor entering at the inlet of the gas cooler ($W_a$) and exiting at the outlet ($W_b$) of the gas cooler are considered for providing the output at step 550. If it is determined that the calculated surface area (A) of the gas cooler 104, 204, 400 not matches with the actual surface area (CA) of the gas cooler 104, 204, 400, then the method 500 repeat the steps from the step 508 to step 548, where, assuming the flow of the gas mixture ($F_a$, should be > exit flow at the outlet 404 of the gas cooler 204) at the inlet 402 of the gas cooler 204, the condensate flow ($F_c$) is calculated using the equation (4), $F_c = F_a - F_b$.

At step 550, if the calculated area (A) matches with the actual surface area (CA) of the gas cooler, then an estimated flow of the gas mixture and condensate, an estimated temperature of the coolant at the outlet 410 and a percentage of the moisture content of the gas mixture at the inlet 402 and outlet 404 of the gas cooler 204 are provided/assigned as the output 216.

An advantage of the above-mentioned system for estimating the moisture content of the gas mixture in the Sample Handling System (SHS), is to avoid the dependency on requirement of hardware based moisture sensors. Another advantage of the system is to improve the reliability of the gas analyzer readings. Yet another advantage of the system is to enable the estimation of the moisture content by providing additional feature (moisture measurement) and without any additional cost (no need of hardware component) to an existing/conventional Sample Handling System.

The system can be applied in the existing Sample Handling Systems and the moisture estimation can be used as one of the measurements while predicting certain types of faults (cooler choking, pump failure) in the Sample Handling System. Further, the system can be integrated with the existing Sample Handling Systems to assist in providing specific types of predictive diagnostic solutions.

## Claims

1.    A system (200) for estimating a moisture content of a gas mixture in a Sample Handling System, SHS (100), the system (200) comprising:

a data pre-processing module (212) configured to:

receive data (211) comprising measurements (M1-M6, S1) of the SHS related to the gas mixture, a gas cooler and a coolant and a plurality of parameters related to the gas mixture ;
analyze the received data to remove one or more anomalies from the data (211) and

a moisture content estimation module (214) configured to:

determine thermodynamic properties, heat transfer coefficient (U) and mass transfer coefficient (K) of the gas mixture based on the analyzed data,
calculate a surface area (A) of the gas cooler based on the analyzed data and the determined thermodynamic properties, heat transfer coefficient (U) and mass transfer coefficient (K) of the gas mixture, and
estimate the moisture content of the gas mixture based on comparison of the calculated surface area (A) of the gas cooler (104, 204, 400) with an actual surface area (CA) of the gas cooler (104, 204, 400).

2. The system (200) as claimed in claim 1, wherein the moisture content estimation module (214) is configured to calculate the surface area of the gas cooler (104, 204, 400) by determining initial temperatures of the gas mixture at the gas cooler (104, 204, 400) of the SHS (100) using computational fluid dynamics finite volume/finite element simulations, and wherein flow of the gas mixture at an inlet (402) of the gas cooler is predefined.

3. The system (200) as claimed in claim 2, wherein the moisture content estimation module (214) is configured to estimate the moisture content of the gas mixture based on comparison of the calculated surface area of the gas cooler (104, 204, 400) with the actual surface area of the gas cooler (104, 204, 400) by determining that the calculated surface area (A) of the gas cooler (104, 204, 400) matches with the actual surface area (CA) of the gas cooler (104, 204, 400), in response to the determination, the moisture content estimation module is configured to provide:

an estimated flow of the gas mixture at the inlet (402) of the gas cooler and condensate,
an estimated temperature of the coolant at an outlet (410) of the coolant, and
the moisture content of the gas mixture at the inlet (402) and an outlet (404) of the gas cooler (104, 204, 400).

4. The system (200) as claimed in claim 2, wherein the moisture content estimation module (214) is configured to iteratively perform the estimation of the thermodynamic properties, the heat transfer coefficient (U) and the mass transfer coefficient (K) of the gas mixture using the analyzed data with a modified value for the flow of the gas mixture at the inlet (402) of the gas cooler (104, 204, 400), in response to the determination that the calculated surface area of the gas cooler (104, 204, 400) not matches with the actual surface area of the gas cooler (104, 204, 400).

5. The system (200) as claimed in claim 1, wherein the data-preprocessing module (212) is configured to analyze the received data to remove one or more anomalies in the data (211) by assigning missing values in the measurements (M1-M6, S1) of the SHS (100), removing noise and outliers in t he data (211) using techniques for imputation, and filtering techniques.

6. The system (200) as claimed in claim 1, wherein the measurements of the SHS (100) related to the gas mixture, the gas cooler and the coolant includes real time data obtained from the SHS (100); wherein the real time data includes pressure, temperature and flow of the gas mixture, the gas cooler and the coolant.

7. The system (200) as claimed in claim 6, wherein the real time data from the gas cooler (104, 204, 400) and the coolant of the SHS (100) includes the temperatures and the flows at the inlet (402) and the outlet (404) of the gas cooler (104, 204, 400) and at an inlet (406) of the coolant.

8. The system (200) as claimed in claim 1, wherein the plurality of parameters related to the gas mixture comprises parameters for determining the thermodynamic properties of the gas mixture and wherein the thermodynamic properties of the gas mixture includes specific heat, heat of condensation, saturated vapor pressure and dynamic viscosity.

9. The system (200) as claimed in claim 1, wherein the plurality of parameters related to the gas mixture comprises a plurality of curves; wherein the plurality of curves comprises Chilton curves, wherein the Chilton curves provide Chilton factor based on Reynolds number of flow, wherein the Chilton factor is utilized for determining the heat transfer coefficient (U) and mass transfer coefficient (K).

10. A method (300) for estimating a moisture content in a gas mixture in a Sample Handling System, SHS, (100), the method comprising:

receiving (302) data (211) comprising measurements of the SHS (100) related to the gas mixture, a gas cooler and a coolant and a plurality of parameters related to the gas mixture,
analyzing the received data to remove one or more anomalies in the data (211),
determining (306) thermodynamic properties, heat transfer coefficient (U) and mass transfer coefficient (K) of the gas mixture based on the analyzed data,
calculating (308) a surface area of the gas cooler based on the analyzed data and the determined thermodynamic properties, heat transfer coefficient (U) and mass transfer coefficient (K) of the gas mixture, and
estimating (310) the moisture content of the gas mixture based on comparison of the calculated surface area (A) of the gas cooler (104, 204, 400) with an actual surface area (CA) of the gas cooler (104, 204, 400) .

**Patentansprüche**

1. System (200) zum Schätzen eines Feuchtigkeitsgehalts eines Gasgemischs in einem Probenhandhabungssystem, SHS (100), wobei das System (200) Folgendes umfasst:
ein Datenvorverarbeitungsmodul (212), das zu Folgendem ausgelegt ist:

Empfangen von Daten (211), die Messungen (M1-M6, S1) des SHS in Bezug auf das Gasgemisch, einen Gaskühler und ein Kühlmittel und mehrere Parameter in Bezug auf das Gasgemisch umfassen;
Analysieren der empfangenen Daten, um eine oder mehrere Anomalien aus den Daten zu entfernen (211), und
ein Feuchtigkeitsgehalt-Schätzmodul (214), das zu Folgendem ausgelegt ist:

Bestimmen von thermodynamischen Eigenschaften, eines Wärmeübertragungskoeffizienten (U) und eines Stoffübergangskoeffizienten (K) des Gasgemischs basierend auf den analysierten Daten,
Berechnen eines Oberflächenbereichs (A) des Gaskühlers basierend auf den analysierten Daten und den bestimmten thermodynamischen Eigenschaften, dem bestimmten Wärmeübertragungskoeffizienten (U) und dem bestimmten Stoffübergangskoeffizienten (K) des Gasgemischs, und
Schätzen des Feuchtigkeitsgehalts des Gasgemischs basierend auf dem Vergleich des berechneten Oberflächenbereichs (A) des Gaskühlers (104, 204, 400) mit einem tatsächlichen Oberflächenbereich (CA) des Gaskühlers (104, 204, 400).

2. System (200) nach Anspruch 1, wobei das Feuchtigkeitsgehalt-Schätzmodul (214) dazu ausgelegt ist, den Oberflächenbereich des Gaskühlers (104, 204, 400) durch Bestimmen von Anfangstemperaturen des Gasgemischs an dem Gaskühler (104, 204, 400) des SHS (100) unter Verwendung von Finite-Volumen-/Finite-Elemente-Simulationen der numerischen Strömungsmechanik zu berechnen, und wobei ein Durchfluss des Gasgemischs an einem Einlass (402) des Gaskühlers vordefiniert ist.

3. System (200) nach Anspruch 2, wobei das Feuchtigkeitsgehalt-Schätzmodul (214) dazu ausgelegt ist, den Feuchtigkeitsgehalt des Gasgemischs basierend auf dem Vergleich des berechneten Oberflächenbereichs des Gaskühlers (104, 204, 400) mit dem tatsächlichen Oberflächenbereich des Gaskühlers (104, 204, 400) durch Bestimmen zu schätzen, dass der berechnete Oberflächenbereich (A) des Gaskühlers (104, 204, 400) mit dem tatsächlichen Oberflächenbereich (CA) des Gaskühlers (104, 204, 400) übereinstimmt,
wobei als Reaktion auf die Bestimmung das Feuchtigkeitsgehalt-Schätzmodul dazu ausgelegt ist, Folgendes bereitzustellen:

einen geschätzten Durchfluss des Gasgemischs an dem Einlass (402) des Gaskühlers und von Kondensat,
eine geschätzte Temperatur des Kühlmittels an einem Auslass (410) des Kühlmittels, und
den Feuchtigkeitsgehalt des Gasgemischs an dem Eingang (402) und einem Ausgang (404) des Gaskühlers (104, 204, 400).

4. System (200) nach Anspruch 2, wobei das Feuchtigkeitsgehalt-Schätzmodul (214) dazu ausgelegt ist, die Schätzung der thermodynamischen Eigenschaften, des Wärmeübertragungskoeffizienten (U) und des Stoffübergangskoeffizienten (K) des Gasgemischs unter Verwendung der analysierten Daten mit einem modifizierten Wert für den Durchfluss des Gasgemischs an dem Einlass (402) des Gaskühlers (104, 204, 400) iterativ als Reaktion auf die Bestimmung durchzuführen, dass der berechnete Oberflächenbereich des Gaskühlers (104, 204, 400) nicht mit dem tatsächlichen Oberflächenbereich des Gaskühlers (104, 204, 400) übereinstimmt.

5. System (200) nach Anspruch 1, wobei das Datenvorverarbeitungsmodul (212) dazu ausgelegt ist, die empfangenen Daten zu analysieren, um eine oder mehrere Anomalien in den Daten (211) durch Zuweisen fehlender Werte in den Messungen (M1-M6, S1) des SHS (100) Entfernen von Rauschen und Ausreißern in den Daten (211) unter Verwendung von Techniken zur Imputation und Filtertechniken zu entfernen.

6. System (200) nach Anspruch 1, wobei die Messungen des SHS (100) in Bezug auf das Gasgemisch, den Gaskühler und das Kühlmittel Echtzeitdaten umfassen, die von dem SHS (100) erhalten wurden; wobei die Echtzeitdaten einen Druck, eine Temperatur und einen Durchfluss des Gasgemischs, des Gaskühlers und des Kühlmittels umfassen.

7. System (200) nach Anspruch 6, wobei die Echtzeitdaten von dem Gaskühler (104, 204, 400) und dem Kühlmittel des SHS (100) die Temperaturen und die Durchflüsse an dem Einlass (402) und dem Auslass (404) des Gaskühlers (104, 204, 400) und an einem Einlass (406) des Kühlmittels umfassen.

8. System (200) nach Anspruch 1, wobei die mehreren Parameter in Bezug auf das Gasgemisch Parameter zum Bestimmen der thermodynamischen Eigenschaften des Gasgemischs umfassen und wobei die thermodynamischen Eigenschaften des Gasgemischs eine spezifische Wärme, eine Kondensationswärme, einen Sättigungsdampfdruck und eine dynamische Viskosität umfassen.

9. System (200) nach Anspruch 1, wobei die mehreren Parameter in Bezug auf das Gasgemisch mehrere Kurven umfassen; wobei die mehreren Kurven Chilton-Kurven umfassen, wobei die Chilton-Kurven einen Chilton-Faktor basierend auf der Reynolds-Zahl des Durchflusses bereitstellen, wobei der Chilton-Faktor zum Bestimmen des Wärmeübertragungskoeffizienten (U) und des Stoffübergangskoeffizienten (K) verwendet wird.

10. Verfahren (300) zum Schätzen eines Feuchtigkeitsgehalts in einem Gasgemisch in einem Probenhandhabungssystem, SHS, (100), wobei das Verfahren Folgendes umfasst:

   Empfangen (302) von Daten (211), die Messungen des SHS (100) in Bezug auf das Gasgemisch, einen Gaskühler und ein Kühlmittel und eine Vielzahl von Parametern in Bezug auf das Gasgemisch umfassen,
   Analysieren der empfangenen Daten, um eine oder mehrere Anomalien in den Daten (211) zu entfernen,
   Bestimmen (306) von thermodynamischen Eigenschaften, eines Wärmeübertragungskoeffizienten (U) und eines Stoffübergangskoeffizienten (K) des Gasgemischs basierend auf den analysierten Daten,
   Berechnen (308) eines Oberflächenbereichs des Gaskühlers basierend auf den analysierten Daten und den bestimmten thermodynamischen Eigenschaften, dem bestimmten Wärmeübertragungskoeffizienten (U) und dem bestimmten Stoffübergangskoeffizienten (K) des Gasgemischs, und
   Schätzen (310) des Feuchtigkeitsgehalts des Gasgemischs basierend auf dem Vergleich des berechneten Oberflächenbereichs (A) des Gaskühlers (104, 204, 400) mit einem tatsächlichen Oberflächenbereich (CA) des Gaskühlers (104, 204, 400).

**Revendications**

1. Système (200) d'estimation d'une teneur en humidité d'un mélange gazeux dans un système de manipulation d'échantillons, SHS (100), le système (200) comprenant :
   un module de prétraitement de données (212) configuré pour :

   recevoir des données (211) comprenant des mesures (M1-M6, S1) du SHS relatives au mélange gazeux, à un refroidisseur de gaz et à un liquide de refroidissement et une pluralité de paramètres relatifs au mélange gazeux ;
   analyser les données reçues pour éliminer une ou plusieurs anomalies des données (211) et
   un module d'estimation de teneur en humidité (214) configuré pour :

   déterminer des propriétés thermodynamiques, un coefficient de transfert de chaleur (U) et un coefficient de transfert de masse (K) du mélange gazeux sur la base des données analysées,
   calculer une superficie (A) du refroidisseur de gaz sur la base des données analysées et des propriétés thermodynamiques, du coefficient de transfert de chaleur (U) et du coefficient de transfert de masse (K) déterminés du mélange gazeux, et
   estimer la teneur en humidité du mélange gazeux sur la base d'une comparaison de la superficie calculée (A) du refroidisseur de gaz (104, 204, 400) avec une superficie réelle (CA) du refroidisseur de gaz (104, 204, 400).

2. Système (200) selon la revendication 1, dans lequel le module d'estimation de teneur en humidité (214) est configuré pour calculer la superficie du refroidisseur de gaz (104, 204, 400) en déterminant des températures initiales du mélange gazeux au niveau du refroidisseur de gaz (104, 204, 400) du SHS (100) à l'aide de simulations de volume fini/d'éléments finis de mécanique des fluides numérique, et dans lequel l'écoulement du mélange gazeux au niveau d'une entrée (402) du refroidisseur de gaz est prédéfini.

3. Système (200) selon la revendication 2, dans lequel le module d'estimation de teneur en humidité (214) est configuré pour estimer la teneur en humidité du mélange gazeux sur la base d'une comparaison de la superficie calculée du refroidisseur de gaz (104, 204, 400) avec la superficie réelle du refroidisseur de gaz (104, 204, 400) en déterminant que la superficie calculée (A) du refroidisseur de gaz (104, 204, 400) correspond à la superficie réelle (CA) du refroidisseur de gaz (104, 204, 400),
en réponse à la détermination, le module d'estimation de teneur en humidité est configuré pour fournir :

un écoulement estimé du mélange gazeux au niveau de l'entrée (402) du refroidisseur de gaz et du condensat, une température estimée du liquide de refroidissement au niveau d'une sortie (410) du liquide de refroidissement, et
la teneur en humidité du mélange gazeux au niveau de l'entrée (402) et d'une sortie (404) du refroidisseur de gaz (104, 204, 400).

4. Système (200) selon la revendication 2, dans lequel le module d'estimation de teneur en humidité (214) est configuré pour réaliser de manière itérative l'estimation des propriétés thermodynamiques, du coefficient de transfert de chaleur (U) et du coefficient de transfert de masse (K) du mélange gazeux à l'aide des données analysées avec une valeur modifiée pour l'écoulement du mélange gazeux au niveau de l'entrée (402) du refroidisseur de gaz (104, 204, 400), en réponse à la détermination que la superficie calculée du refroidisseur de gaz (104, 204, 400) ne correspond pas à la superficie réelle du refroidisseur de gaz (104, 204, 400).

5. Système (200) selon la revendication 1, dans lequel le module de prétraitement de données (212) est configuré pour analyser les données reçues afin d'éliminer une ou plusieurs anomalies dans les données (211) en attribuant des valeurs manquantes dans les mesures (M1-M6, S1) du SHS (100), éliminant le bruit et les valeurs aberrantes dans les données (211) à l'aide de techniques d'imputation, et de techniques de filtrage.

6. Système (200) selon la revendication 1, dans lequel les mesures du SHS (100) relatives au mélange gazeux, au refroidisseur de gaz et au liquide de refroidissement incluent des données en temps réel obtenues en provenance du SHS (100) ; les données en temps réel incluant la pression, la température et l'écoulement du mélange gazeux, du refroidisseur de gaz et du liquide de refroidissement.

7. Système (200) selon la revendication 6, dans lequel les données en temps réel en provenance du refroidisseur de gaz (104, 204, 400) et du liquide de refroidissement du SHS (100) incluent les températures et les écoulements au niveau de l'entrée (402) et de la sortie (404) du refroidisseur de gaz (104, 204, 400) et au niveau d'une entrée (406) du liquide de refroidissement.

8. Système (200) selon la revendication 1, dans lequel la pluralité de paramètres relatifs au mélange gazeux comprend des paramètres pour déterminer les propriétés thermodynamiques du mélange gazeux et les propriétés thermodynamiques du mélange gazeux incluant chaleur spécifique, chaleur de condensation, pression de vapeur saturée et viscosité dynamique.

9. Système (200) selon la revendication 1, dans lequel la pluralité de paramètres relatifs au mélange de gaz comprend une pluralité de courbes ; la pluralité de courbes comprenant des courbes de Chilton, les courbes de Chilton fournissant un facteur de Chilton sur la base du nombre de Reynolds d'écoulement, le facteur de Chilton étant utilisé pour déterminer le coefficient de transfert de chaleur (U) et le coefficient de transfert de masse (K).

10. Procédé (300) d'estimation d'une teneur en humidité dans un mélange gazeux dans un système de manipulation d'échantillons, SHS, (100), le procédé comprenant :

la réception (302) de données (211) comprenant des mesures du SHS (100) relatives au mélange gazeux, au refroidisseur de gaz et à un liquide de refroidissement et une pluralité de paramètres relatifs au mélange gazeux, l'analyse des données reçues pour éliminer une ou plusieurs anomalies dans les données (211),

la détermination (306) de propriétés thermodynamiques, d'un coefficient de transfert de chaleur (U) et d'un coefficient de transfert de masse (K) du mélange gazeux sur la base des données analysées,

le calcul (308) d'une superficie du refroidisseur de gaz sur la base des données analysées et des propriétés thermodynamiques, du coefficient de transfert de chaleur (U) et du coefficient de transfert de masse (K) déterminés du mélange gazeux, et

l'estimation (310) de la teneur en humidité du mélange gazeux sur la base d'une comparaison de la superficie calculée (A) du refroidisseur de gaz (104, 204, 400) avec une superficie réelle (CA) du refroidisseur de gaz (104, 204, 400).

100

101 Flue gas stack

102 Sample Probe
Ring Heater and Ceramic Filter

103 Heat tracer line

Ring heater temperature (M1)

Heat tracer line temperature (M2)

Cooler inlet pressure (M3)

104 Gas Cooler

Cooler Temperature (M4)

Condensate (112)

Cooler outlet pressure (M5)

106 Pump

Flow measurement (S1)

Pump discharge pressure (M6)

Bypass flow

108 Gas Analyzer

110 Gas

Fig. 1

Fig. 2

300

Receiving data comprising a measurements from a SHS, and a plurality of parameters — 302

Processing the data to remove one or more anomalies from the data — 304

Determining thermodynamic properties, heat transfer coefficient (U) and mass transfer coefficient (K) of the gas mixture — 306

Calculating a Surface area of the gas cooler — 308

Estimating the moisture content of the gas mixture — 310

Fig. 3

Fig. 4

500

| Calculate saturated water vapor pressures at an inlet and outlet of the gas cooler | 502 |

↓

| Calculate gaseous vapor pressures at the inlet and outlet of the gas cooler | 504 |

↓

| Calculate amount of water vapor and gases at the outlet of the gas cooler | 506 |

B →

| Calculate a condensate flow Fc | 508 |

↓

| Calculate amount of the water vapor and gases at the inlet of the gas cooler | 510 |

↓

| Calculate heat transferred in condensing and cooling of the Fc moles of the water vapor | 512 |

↓

| Calculate the heat transferred in cooling non-condensable gas mixture | 514 |

↓

| Calculate the heat transferred in cooling un-condensed water vapor | 516 |

↓

| Calculate temperature (Te) at the outlet of the coolant | 518 |

↓

| Calculate mass velocity of the gases and the vapor mixture entering the gas cooler | 520 |

↓

| Calculate Reynolds number for the entering gases and the vapor mixture | 522 |

↓

| Calculate heat transfer factor (j) from the Reynolds number (Re) | 524 |

↓

| Calculate heat transfer coefficient in a gas film | 526 |

↓

| Calculate rate of diffusion of vapor molecules through the gas film | 528 |

↓

A

Fig. 5A

500

A

Divide a temperature range of the gas cooler into ten points grid — 530

Calculate a log mean of the non-condensable gas vapor pressure — 532

Calculate rate of the heat transfer through gas and condensate films — 534

Calculate rate of the heat transfer through tubes and coolant film — 536

Compare the value obtained at step 534 and step 536 — 538

No — Equal — 540

Yes

Calculate over-all heat transfer coefficient (U) — 542

Calculate a surface area (A) of the gas cooler — 544

Compare the calculated surface area (A) with an actual surface area (CA) — 546

B — No — Equal — 548

Yes

Provide an estimated flow of the gas mixture and condensate, an estimated temperature of the coolant and the moisture content of the gas mixture at the gas cooler as output — 550

Fig. 5B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020053807 A1